(19) Europäisches Patentamt
European Patent Office
Office européen des brevets

(11) Numéro de publication: **0 564 568 B1**

# (12) FASCICULE DE BREVET EUROPEEN

(45) Date de publication de fascicule du brevet: **02.11.94**

(51) Int. Cl.5: **C07B 35/02**, C07F 15/00, C07C 51/36

(21) Numéro de dépôt: **92903463.5**

(22) Date de dépôt: **27.12.91**

(86) Numéro de dépôt internationale :
**PCT/FR91/01077**

(87) Numéro de publication internationale :
**WO 92/12110 (23.07.92 92/19)**

## (54) PROCEDE D'HYDROGENATION DE COMPOSES ORGANIOUES ETHYLENIOUEMENT INSATURES ET CATALYSEURS AU RUTHENIUM POUR SA REALISATION.

(30) Priorité: **28.12.90 FR 9016414**

(43) Date de publication de la demande:
**13.10.93 Bulletin 93/41**

(45) Mention de la délivrance du brevet:
**02.11.94 Bulletin 94/44**

(84) Etats contractants désignés:
**AT BE CH DE DK ES FR GB GR IT LI LU MC NL SE**

(56) Documents cités:
**EP-A- 0 272 787**
**EP-A- 0 366 390**

**JOURNAL OF ORGANIC CHEMISTRY, vol. 52, No. 14, 10 July 1987, EASTON US, pages 3174-3176; T: OHTA ET AL.: "ASYMMETRIC HYDROGENATION OF UNSATURATED CARBOXYLIC ACIDS CATALYZED BY BINAP-RUTHENIUM (II) COMPLEXES", cited in the application, see the whole document**

(73) Titulaire: **ELF AOUITAINE**
**Tour Elf,**
**2, Place de la Coupole,**
**La Défense 6**
**F-92400 Courbevoie (FR)**

(72) Inventeur: **GENET, Jean-Pierre**
**70, rue Estienne-d'Orves**
**F-91370 Verrières-le-Buisson (FR)**
Inventeur: **JUGE, Sylvain 15, sentier de Chateaufort**
**"Le Champ des Cordes"**
**F-91400 Orsay (FR)**
Inventeur: **LAFFITE, Jean, Alex**
**60, boulevard des Pyrénées**
**F-64000 Pau (FR)**
Inventeur: **PINEL, Catherine "La Brasserie"**
**Orson Ville**
**F-78660 Ablis (FR)**
Inventeur: **MALLART, Serge**
**8, avenue des Chênes**
**F-91400 Orsay (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

JOURNAL OF THE CHEMICAL SOCIETY, SECTION A 1971, London, pages 16-20; M. COOKE ET AL.: "PI-ALLYLIC COMPLEXES OF RUTHENIUM"

(74) Mandataire: **Bertrand, Didier et al**
**c/o S.A. Fedit-Loriot**
**38, avenue Hoche**
**F-75008 Paris (FR)**

## Description

L'invention se rapporte à l'hydrogénation de composés organiques éthylèniquement insaturés présentant au moins une double liaison C=C aliphatique. Elle comprend un procédé permettant d'effectuer avantageusement une telle opération en milieu homogène, en présence d'un catalyseur formé par un complexe de ruthénium. En outre, elle vise un système catalytique du Ru avec un ligand phosphoré, capable de produire une hydrogénation asymétrique, avec excellent rendement et excès énantiomérique.

On sait que des complexes neutres du ruthénium de structure bis(2-méthylallyl)-bis(phosphite)-ruthénium ont été décrits dans l'article de M. COOKE et al. J. Chem. Soc. (A), pages 16-20 (1971). Il s'agit plus précisément de complexes répondant à la formule

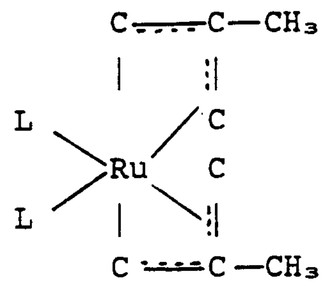

où

- composé (C1) : L = $(MeO)_3P$
- composé (C2) : L = $(EtO)_3P$
- composé (C3) : L = $MeC(CH_2O)_3P$
- composé (C4) : L = $EtC(CH_2O)_3P$
- composé (C5) : L = $Me_2PCH_2CH_2PMe_2$

Il se trouve que ledit article de M. COOKE et al., ne décrit ni ne suggère l'utilisation de ces cinq composés dans le domaine de l'hydrogénation catalytique de composés éthylèniquement insaturés.

On sait par ailleurs que des complexes ioniques du ruthénium ont été préconisés dans le passé pour l'hydrogénation catalytique d'oléfines. Voir à cet effet EP-A-0 366 390 qui recommande dans ce but l'utilisation de complexes de formule

$[RuX_l(L)_m(R\text{-}BINAP)]Y_n$ (C6)

où

BINAP est un reste 2,2'-bis(diphénylphosphino)-1,1'binaphtyle, R est H ou $CH_3$ en position 4 de chaque noyau phényle du fragment bis(diphénylphosphino),

X est F, Cl, Br ou I,

L représente notamment le benzène, le p-cymène ou l'acétonitrile,

Y représente un atome d'halogène, $ClO_4$, $PF_6$, $BF_4$, $BPh_4$, avec

- quand L est différent de l'acétonitrile, l = 1, m = 1 et n = 1,
- quand L est acétonitrile, si l = 1, alors m = 2 et n = 1, et si l = O, alors m = 4 et n = 2,

d'une part, et EP-A-O 272 787 qui recommande l'utilisation des complexes de formule

$Ru_xH_yCl_z(R^4\text{-}BINAP)_2(S)_p$ (C7)

où

BINAP est défini comme ci-dessus dans la formule C6,

$R^4$ a la même définition que R ci-dessus dans la formule C6,

S est une amine tertiaire,

quand y = O, alors x = 2, z = 4 et p = 1, et

quand y = 1, alors x = 1, z = 1 et p = O,

d'autre part.

On connaît par ailleurs de l'article de T. OHTA et al., J. Org. Chem., 52, pages 3174-3176, (1987) des catalyseurs neutres du ruthénium pour l'hydrogénation d'acides carboxyliques éthylèniquement insaturés en alpha, béta ou en béta, gamma. Ces catalyseurs qui sont de configuration S ou R sont représentés par les

formules

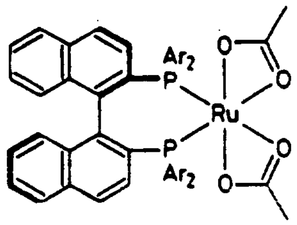 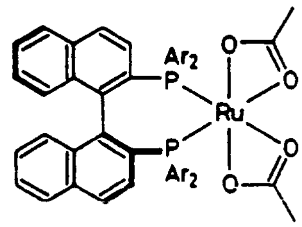

(*R*) (*S*)

a, Ar = $C_6H_5$

b, Ar = *p*-$CH_3C_6H_4$

c, Ar = *p*-$CH_3OC_6H_4$

Il existe un besoin d'une nouvelle solution technique pour l'hydrogénation catalytique de composés organiques éthylèniquement insaturés, qui soit différente des solutions techniques préconisées par les documents EP-A-0 366 390, EP-A-0 272 787 et l'article de T. OHTA et al. précités.

La nouvelle solution technique qui a été recherchée repose sur l'utilisation de complexes neutres du ruthénium analogues par leur structure au composé C5 selon l'article de M. COOKE et al.

Selon un premier aspect de l'invention, on préconise une nouvelle solution technique pour l'hydrogénation catalytique de composés organiques éthylèniquement insaturés comprenant au moins une double liaison C = C aliphatique. En effet, il n'a jamais été question d'utiliser jusqu'à présent comme catalyseur un composé porteur de ligands allyliques. On peut voir, dans les références indiquées plus haut, et dans bien d'autres, différents complexes de Ru avec des phosphines utilisés pour l'hydrogénation en milieu homogène des liaisons C = C, sans qu'aucun des catalyseurs ne comporte un ligand allylique.

Contrairement à cet état des choses, la présente invention résulte de la constatation surprenante que des complexes phosphinés du Ru, dans lesquels Ru porte deux ligands allyliques, peuvent catalyser très efficacement l'hydrogénation asymétrique des liaisons C = C.

Ainsi, la présente invention comprend-elle un procédé d'hydrogénation de composés organiques éthylèniquement insaturés, en milieu homogène, avec, comme catalyseur, un complexe (diphosphino)-ruthénium, caractérisé en ce que, dans ce catalyseur, un atome de ruthénium porte deux ligands formés par des groupes allyliques.

Selon un second aspect de l'invention on préconise en tant que produits industriels nouveaux, utiles dans le domaine de l'hydrogénation catalytique des oléfines et d'une manière générale des composés organiques éthylèniquement insaturés, les composés de Formule (1) ci-après où Q est différent de $CH_2CH_2$ quand $R^1 = R^2 = R^3 = R^4$ = méthyle.

Le catalyseur, utilisé suivant l'invention pour l'hydrogénation catalytique de composés éthylèniquement insaturés et qui appartient à l'ensemble des composés diallyliques de (diphosphino)ruthénium, peut être représenté par la formule schématique

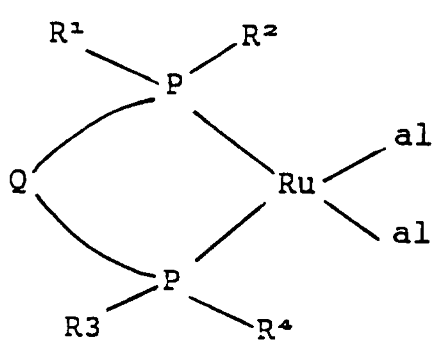

(1)

dans laquelle

- al représente un groupe allylique,
- Q représente un pont hydrocarboné comportant au moins deux atomes de carbone caténaires et susceptible de contenir un à quatre hétéroatomes caténaires choisis parmi O, S, N et Si
- $R^1$ à $R^4$ qui peuvent être identiques ou différents, représentent chacun un groupe alkyle en $C_1$-$C_{18}$, cycloalkyle en $C_5$-$C_7$ ou aryle en $C_6$-$C_{12}$.

Les groupes allyliques al précités comprennent ici tous les groupes allyle, les plus simples et les plus économiques étant plus précisément le groupe allyle proprement dit de formule $CH_2CH=CH_2$ et le groupe méthallyle de formule $CH_2C(CH_3) = CH_2$. Ainsi de façon avantageuse le groupe allylique al sera choisi parmi l'ensemble constitué par les groupes allyle et méthallyle. Quand ils sont liés à l'atome de ruthénium lesdits groupes allyle et méthallyle présentent les configurations $al_2Ru$ suivantes :

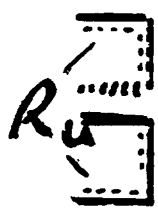

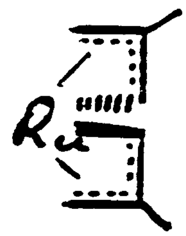

où la double liaison allylique C=C est délocalisée.

Le pont Q est une chaîne comprenant de 2 à 10 atomes caténaires, un ou plusieurs fragments de cette chaîne pouvant être inclus dans au moins un cycle. Quand un atome d'azote caténaire est présent dans ledit pont Q, cet atome d'azote sera ternaire, par exemple : $-N(CH_3)-$, $-N(i-C_3H_7)-$, $-N(t-C_4H_9)-$, $-N(C_6H_5)-$,$-N(CH_2C_6H_5)-$. Quand un atome de silicium caténaire est présent dans ledit pont Q, cet atome de silicium sera quaternaire, par exemple :

$-Si(CH_3)_2-$ , $-Si(CH_2CH_3)_2-$,

$-Si(C_6H_5)_2-$, $-Si(CH_2C_6H_5)-$,

De préférence le pont Q renfermera de 2 à 4 atomes caténaires et aura avantageusement l'une des structures suivantes :

(a)

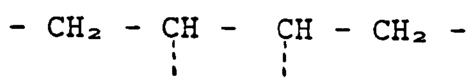

(b) $- CH_2 - CH_2 - CH_2 - CH_2 -$

(c)

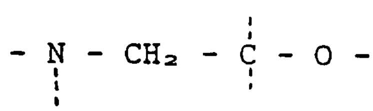

(d)

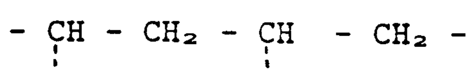

(e)

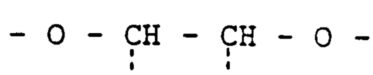

(f)

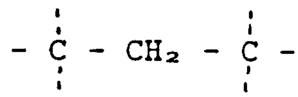

(g)

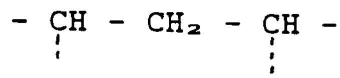

(h)

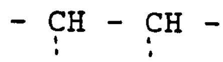

(i) - $CH_2$ - $CH_2$ -
(j)

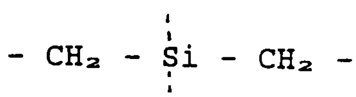 ou

(k)

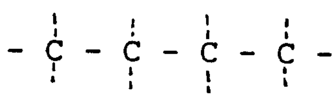

(l) -$CH(CH_3)$-$CH(CH_3)$-
dans lesquelles les traits en pointillé désignent chacun un substituant différent de H, voire encore une double liaison C=C sur deux atomes de carbone vicinaux inclus dans un cycle aryle ; ainsi quand la structure $R^1R^2P$-Q-$PR^3R^4$ représente le groupe (+)-BINAP ou (-)-BINAP, la structure (k) ci-dessus du pont Q peut être :

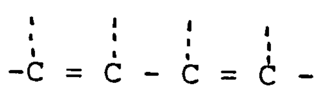

Comme indiqué ci-dessus les groupes $R^1$, $R^2$, $R^3$ et $R^4$, identiques ou différents, représentent chacun un groupe alkyle en $C_1$-$C_8$ (notamment méthyle, éthyle, isopropyle, propyle, butyle, s-butyle, i-butyle, t-butyle, 2,2-dimé-thylpropyle ou 1,1, 3,3-tétraméthylbutyle), un groupe cycloalkyle en $C_5$-$C_7$ (notamment cyclopentyle ou mieux cyclohexyle) ou un groupe aryle en $C_6$-$C_{12}$ (notamment phényle, tolyle, xylyle, p-méthoxyphényle, p-éthoxy-phényle, p-(t-butyloxy)phényle ou naphtyle).

De préférence $R^1$, $R^2$, $R^3$ et $R^4$, identiques ou différents, représenteront chacun un groupe cyclohexyle, phényle, phényle substitué en position para par un groupe alkyle en $C_1$-$C_4$, phényle substitué en position para par un groupe alkoxy en $C_1$-$C_4$, ou 2-naphtyle.

La formule (1) telle que définie ci-dessus englobe le composé C5 décrit par M. COOKE et al. (Q = $CH_2CH_2$ ; $R^1$ = $R^2$ = $R^3$ = $R^4$ = $CH_3$).

A l'exception dudit composé C5 tous les autres caralyseurs répondant à la formule (1) sont nouveaux.

En dehors de la chiralité due aux groupes bidentates, il est possible d'en apporter une par un choix approprié de la partie P-Q-P de la molécule ; on peut notamment l'avoir sur le phosphore, la formule (1)

ayant alors la configuration :

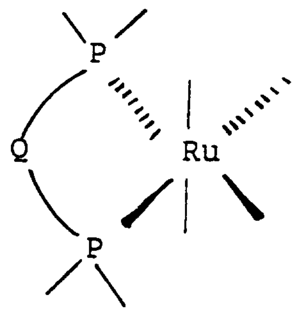

(2)

La préparation des complexes de $al_2Ru$ est connue en soi : elle est décrite dans l'article de M. COOKE et al. précité ainsi que dans la demande de brevet français No 89 11 159 du 23 août 1989 (publication FR-A-2 651 152 du 1 mars 1991).

On rappelle seulement ici qu'un mode opératoire pratique comprend la réaction d'un sel de Ru, par exemple $RuCl_3.3H_2O$, avec un cycladiène, en particulier le cyclooctadiène. Le complexe, ainsi obtenu, est mis à réagir avec un organo-magnésien de formule XMg-al (où al représente le groupe allyle ou méthallyle, comme indiqué ci-dessus et X est F, Cl, Br ou I) pour former un complexe cyclooctadiène-Ru($al_2$). Ce dernier est alors chauffé, en général vers 50° à 70° C pendant plusieurs heures, dans un solvant approprié, avec une diphosphine,

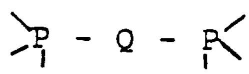

pour former le complexe (1) ou (2).

De façon avantageuse ce procédé sera mis en oeuvre avec un organo-magnésien XMg-al dans lequel X sera Cl et al sera le groupe méthallyle [i.e. $CH_2 = C(CH_3)CH_2$ MgCl].

En tant que diphosphines on peut utiliser les différents composés, connus dans l'art sous des abréviations telles que "DIOP", "CHIRAPHOS", "NORPHOS", "BNPE", "BINAP", "DIPAMP", "BDPP" etc., qui remplacent le cycloalcadiène pour former le catalyseur asymétrique.

De telles dénominations étant utilisées dans les exemples donnés plus loin, on en précise la composition ci-après, par des formules :

(3)

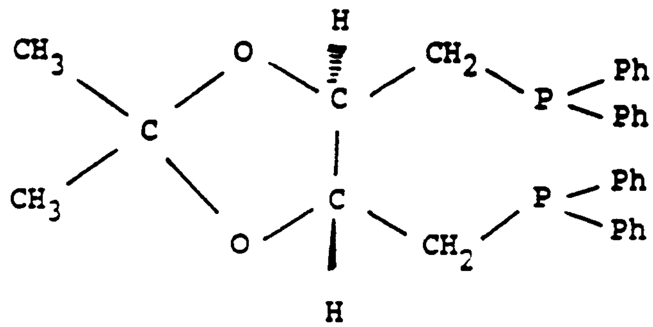

"DIOP"

(4)

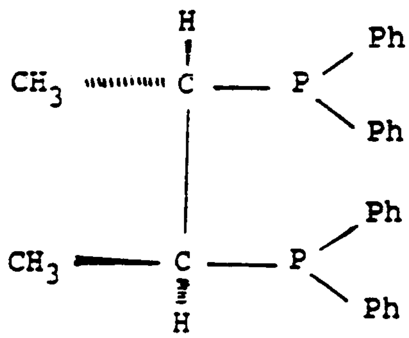

"CHIRAPHOS"

(5)

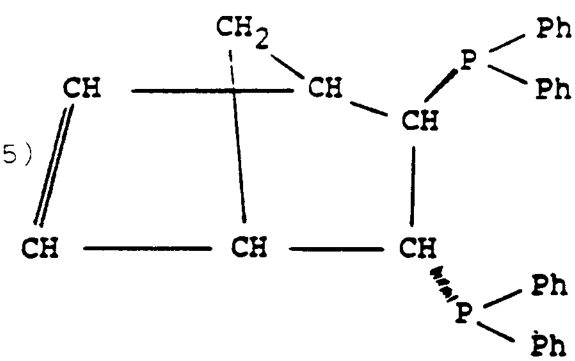

"NORPHOS"

(6)

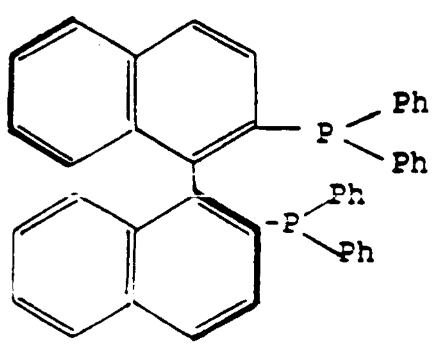

"BINAP"

(7) 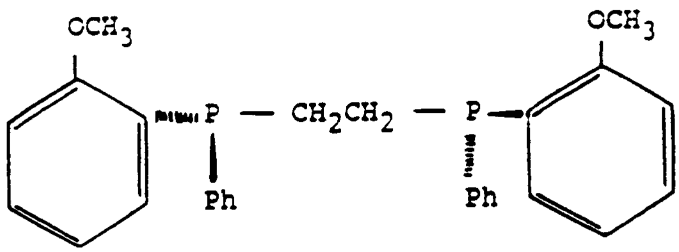 "DIPAMP"

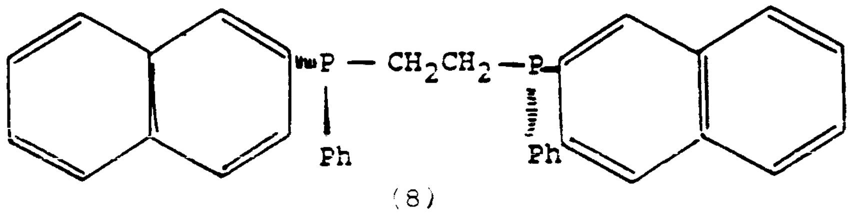 " BNPE"

(8)

(9) 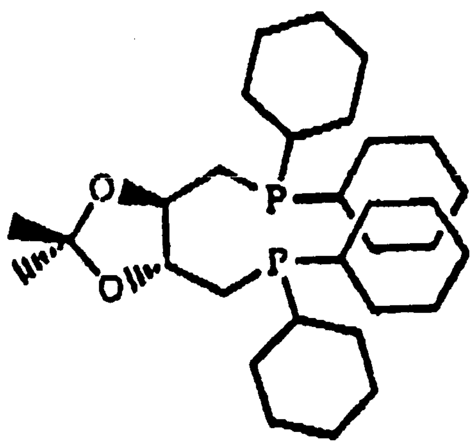 "CyDiOP"

(10) 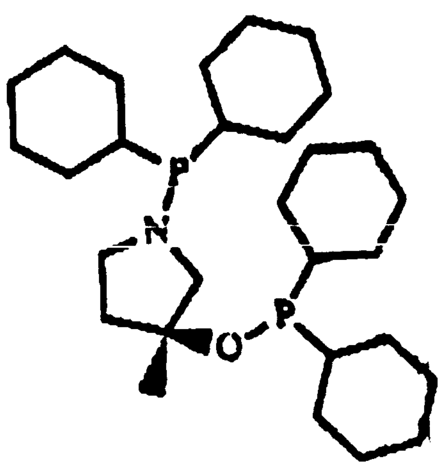 "CyPRONOP"

(11) 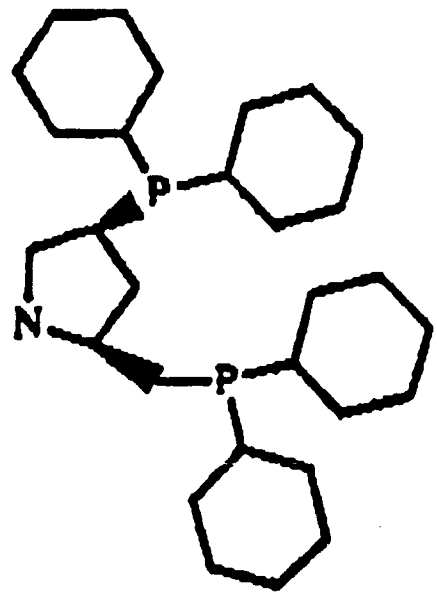 "Cy-cy CAPP"

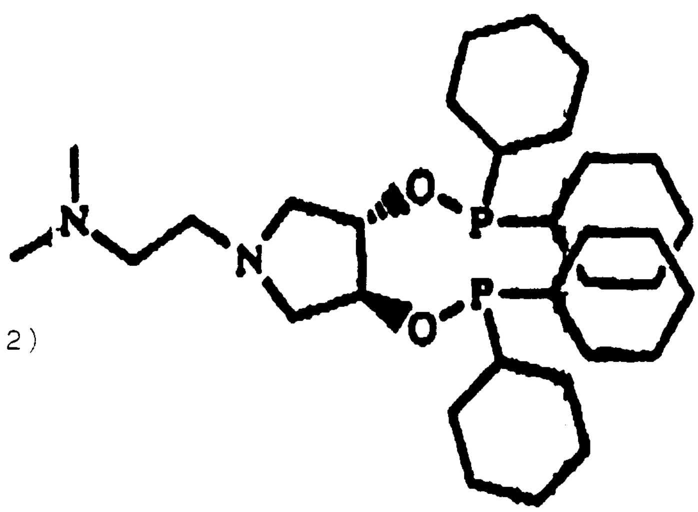 "CyPOP AE"

(12)

Ces phosphines précitées sont mentionnées ici de façon non limitative.

Grâce aux conditions douces de phosphination, indiquées plus haut, que l'on applique dans la préparation des nouveaux catalyseurs, diverses mono- ou di-phosphines tertiaires, porteuses de chiralité sur l'atome de P, peuvent être obtenues, sans que les ligands du complexe subissent une racémisation qui devient sensible au-dessus de 80 ° C.

Contrairement à la technique antérieure, qui n'utilisait pratiquement que la diphosphine "BINAP", assez coûteuse, pour arriver à hydrogéner des doubles liaisons C = C (MASATO KITAMURA, J. Org. Chem. 1987, 52 pages 3174-3176), la présente invention permet de changer facilement de ligands : cela concerne particulièrement les ligands porteurs de chiralité sur le phosphore. Comme les exemples le montrent,

l'invention permet l'hydrogénation asymétrique, économique, des liaisons oléfiniques, avec des catalyseurs au Ru ayant différents ligands.

L'invention est illustrée par les exemples non limitatifs, donnés plus loin, sur l'hydrogénation de la double liaison oléfinique, $>C=C<$ dans des acides carboxyliques. Ainsi, une oléfine peut être réduite, suivant l'invention, lorsqu'elle porte une ou plusieurs fonctions.

Suivant la présente invention, l'hydrogénation est en général réalisée dans une solution de 0,1 à 3 moles de composé insaturé à traiter par litre d'un solvant approprié, qui peut être, selon les cas, par exemple un alcool, notamment méthanol, éthanol, propanol, etc. anhydre, ou un hydrocarbure tel que benzène, toluène, xylène, pentane, hexane, heptane et similaire, éventuellement un mélange de tels solvants. Dans le solvant on dissout une quantité de catalyseur de 0,1 à 5 % en moles par rapport au composé insaturé. Conviennent en général bien des proportions de catalyseur d'environ 0,5 à 2 moles %.

Dans les exemples qui suivent, le mode opératoire de l'hydrogénation était exécuté comme indiqué ci-après. Dans un réacteur à atmosphère d'argon on introduit 1 mmole de substrat oléfinique à hydrogéner et 3 ml de méthanol sec, dégazé. On y ajoute 1 % molaire de catalyseur constitué par un complexe phosphiné de Ru. L'atmosphère d'argon est alors remplacée par de l'hydrogène et l'on établit la pression de ce dernier à une valeur de 1 à 100 bars. Le mélange est agité durant 24 à 64 heures à 20° C. L'expérience a montré d'ailleurs que les températures préférées pour de telles hydrogénations s'échelonnent entre 0° et 50°C.

Après l'évaporation du solvant, le taux de conversion du produit oléfinique en composé saturé correspondant est déterminé par RMN.

Les exemples, dont les résultats sont donnés ci-après, correspondent à l'utilisation des catalyseurs suivant l'invention, comportant deux groupes méthallyle liés à l'atome de Ru.

<u>Exemples 1 à 7</u>

Suivant le mode opératoire précisé plus haut, on effectue l'hydrogénation de l'acide 2-méthyl-2-butènoique,

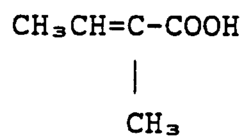

en acide 2-méthylbutanoique, soit

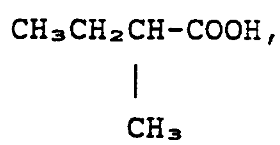

à 20°C, sous différentes pressions et pendant des durées variables.

Le catalyseur DIOP-Ru(Méthallyl)$_2$ présentait les caractéristiques suivantes :

$^1$H RNM (250 MHz, $C_6D_6$) : 1,0 (m, 2H) ; 1,3 (s, 6H) ; 1,32 (q,J = 14,5 Hz, 4H) ; 2,04 (s, 6H) ; 2,55 (m, 2H) ; 2,79 (dd,$J_1$ = 8,5 Hz, $J_2$ = 13 Hz, 6H) ; 3,25 (t,J = 13 Hz, 6H) ; 4,15 (m, 2H) ; 6,8-8,0 (m,20H, aromatiques).

$^{13}$C RMN (62 MHz, $C_6D_6$) : 25,77 ; 27,2 ; 31,5 (m) ; 42,5 ; 48,31 (m) ; 78,8 ; 95,7 ; 107,9 ; 127-140 (aromatiques).

$^{31}$P RMN (100 MHz, $C_6D_6$) : 36 (ref. $H_3PO_4$ 75 %)

IR (Nujol) : 1595, 1240 cm$^{-1}$

$\alpha_D^{25}$ = + 202° (c = 0,43, toluène) ; fusion 204°C (décomposition).

Celles du catalyseur CHIRAPHOS-Ru(Méthallyl)$_2$ étaient :

$^1$H RMN (250 MHz, $C_6D_6$) : 1,06 (d,J = 6,5 Hz, 2H) ; 1,12 (d,J = 6,5 Hz, 2H) ; 1,24 (q,J = 2,5 Hz, 6H); 1,61 (d,J = 2,5 Hz, 2H) ; 1,74 (d,J = 2,5 Hz, 2H) ; 2,15 (J,6H) ; 6,8-8,0 (m, 20H, aromatiques).

IR (Nujol) : 1580, 1085, 1015, 760, 720 cm$^{-1}$.

$\alpha_D^{25}$ = + 60° (c = 0,2, toluène) ; fusion 183°C (décomposition).

(voir tableau 1 ci-après).

Exemple 8

Hydrogénation de l'acide 2-méthyl-2-hexènoïque de formule

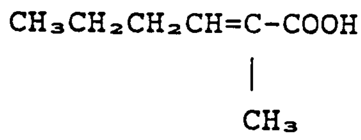

en acide 2-méthylhexanoïque de formule

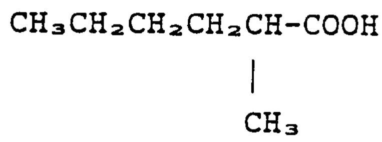

à 20 ° C, sous une pression d'hydrogène de 15 bars, pendant 24 heures.

Le catalyseur CHIRAPHOS-Ru(Méthallyl)$_2$ donne avec un rendement de 100 %, un excès énantiomérique (ee) de 17%.

Exemple 9

Hydrogénation de l'acide 3-phényl-2-méthyl-2-propènoïque de formule

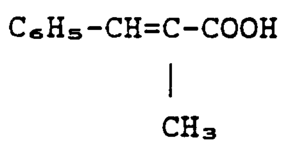

en acide 3-phényl-2-méthylpropanoïque de formule :

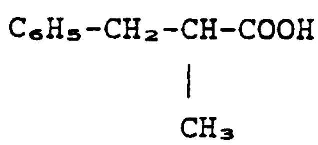

On opère à 20 ° C sous une pression d'hydrogène de 95 bars pendant 24 heures. Le catalyseur CHIRAPHOS-Ru(-)(Méthallyl)$_2$ donne l'acide saturé avec un rendement de 70 %.

Exemple 10

Hydrogénation de l'acide 2-(6-méthoxy-2-naphtyl)-2-propènoïque en acide 2-(6-méthoxy-2-naphtyl)-propionïque, connu en pharmacie sous la dénomination de "NAPROXENE", jouissant de propriétés anti-inflammatoires.

L'opération a lieu à la température ambiante, sous une pression d'hydrogène de 30 bars, pendant 64 heures, en présence du complexe

DIPAMP-Ru (Méthallyl)$_2$

comme catalyseur et 10 % de triéthylamine.

La réaction

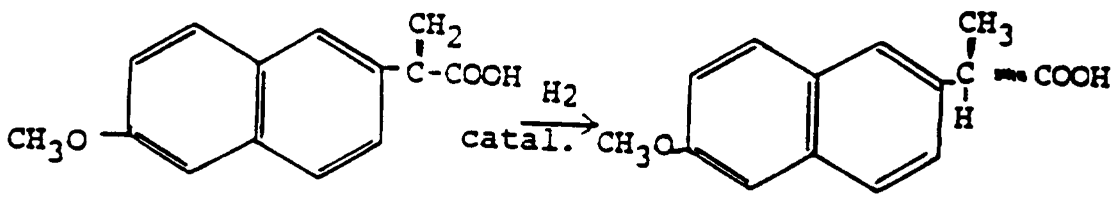


"NAPROXENE"

donne un rendement de 100 %, et ce dernier présente un ee de 55 %.

Voici les caractéristiques du catalyseur DIPAMP-Ru (Méthallyl)$_2$ employé :

$^1$H NMR (250 MHz) : 0,25(d,J=15, 2H) ; 1,1 (dd, $J_1$=15, $J_2$=5, 2H) ; 1,7 (s, 2H) ; 2,2 (d, J = 2Hz, 2H) ; 2,31 (s, 6H) ; 2,92 (s, 6H) ; 3,4 (m, 2H) ; 6,5-8,1 (m, 18H)

$^{13}$C RMN ($CDCl_3$) 26,6 ; 32,5 (dd, $J_1$=$J_2$=27Hz) ; 42,4(d,J = 25 Hz) ; 44,3 ; 54,6 ; 96,1 ; 110,6 ; 120,6 ; 126-130 ; 134,6 ; 142,3 ; 159,9

Couleur jaune F = 183-185° (décomposition)

$/\alpha/_D$ = -43,5° (c = 0,23, toluène)

Exemple 11

Une hydrogénation en tous points similaire à celle de l'exemple 10 est effectuée avec, comme catalyseur, le complexe DIPAMP-Ru (AcO)$_2$. Le rendement est encore de 100 % mais ee est seulement de 22 %, ce qui montre l'avantage des groupes allyliques dans le catalyseur de l'exemple 10.

Il peut être, dans certains cas, avantageux d'utiliser conjointement un complexe de Ru à ligands allyliques avec un complexe correspondant, sans groupes allyliques ; les proportions relatives préférées sont alors de 10 à 90 parties en poids de l'un pour 90 à 10 parties de l'autre.

## TABLEAU I

Hydrogénation de $CH_3-CH = \underset{\displaystyle CH_3}{\overset{}{C}} - COOH$ à 20°C

| EX.N° | CATALYSEUR | Pression (bars) | Temps (h) | Rendem[t] % | ee% | Configu-ration |
|---|---|---|---|---|---|---|
| 1 | DIOP-Ru(-)(Méthallyl)$_2$ | 50 | 24 | 100 | 46 | R |
| 2 | " " " | 15 | " | 100 | 46 | R |
| 3 | " " " | 5 | " | 100 | 48 | R |
| 4 | " " " | 1,5 | 36 | 100 | 51 | R |
| 5 | CHIRAPHOS-Ru(-)(Méthallyl)$_2$ | 15 | 24 | 100 | 30 | S |
| 6 | NORPHOS-Ru(+)(Méthallyl)$_2$ | 1,5 | 60 | 100 | 4 | S |
| 7 | BINAP-Ru(+)(Méthallyl)$_2$ | 15 | 24 | 100 | 87 | R |

Exemple 12

Hydrogénation de l'acide α-acétamido-2-phényl-2-propènoïque de formule

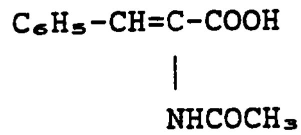

en N-acétyl-phénylalanine de formule :

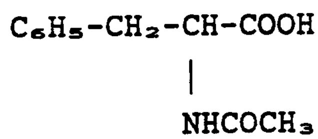

On opère à 20 °C sous une pression d'hydrogène de 100 bars pendant 24 heures, le catalyseur étant le DIPAMP-Ru (Méthallyl)$_2$. La N-acétyl-phénylalanine est obtenue avec un rendement de 70 %.

Exemples 13 à 21

On a préparé 9 complexes chiraux du ruthénium de formule (1) selon l'invention, à partir du chlorure de ruthénium trihydraté et du cyclooctadiène, suivant les mécanismes réactionnels suivants où Mét représente le groupe méthallyle.

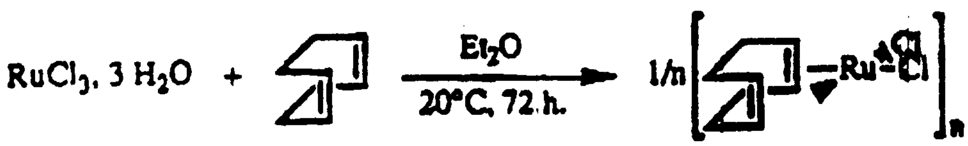

(I)

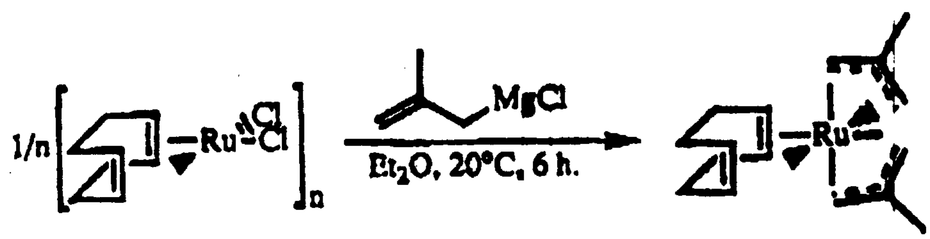

(II)

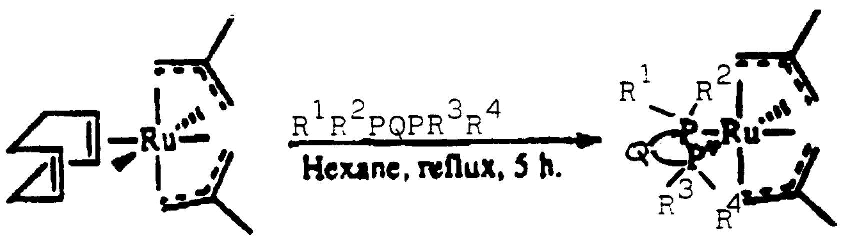

(III)

Les composés chiraux obtenus à partir de différentes diphosphines selon l'équation III sont représentés dans le tableau II ci-après.

TABLEAU II

| LIGAND | | COMPLEXE |
|---|---|---|
| EX. 13 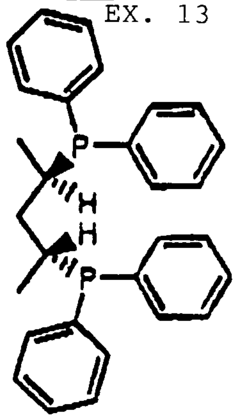 | (-) BDPP<br>*(2S,4S)-2,4-bis(diphénylphosphino) pentane(S,S)*<br>M = 440,50 | **(-) BDPP Ru $(Mét.)_2$**<br>**M = 649,76**<br>**(Vert)**<br>**Rdt = 60%** |

Tableau II (suite)

| | LIGAND | | COMPLEXE |
|---|---|---|---|
| Ex. 14 | 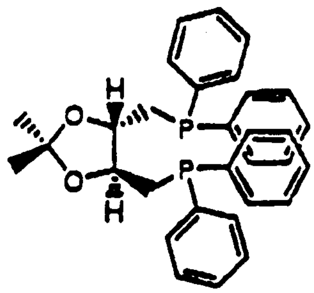 | (+) DIOP<br>*(S,S)-O,O-isopropylidène-2,3-dihydroxy-1,4-bis(diphénylphosphino) butane.*<br>M = 498,54 | (+) DIOP Ru (Mét.)$_2$<br>M = 707,80<br>(Jaune)<br>Rdt = 90% |
| Ex. 15 | 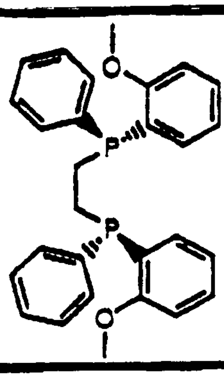 | (-) DIPAMP<br>*(R,R)-1,2-bis(phényl orthoanisylphosphino) éthane.*<br>M = 458,48 | (-) DIPAMP Ru (Mét.)$_2$<br>M = 667,74<br>(Jaune)<br>Rdt = 40%. |
| Ex. 16 | 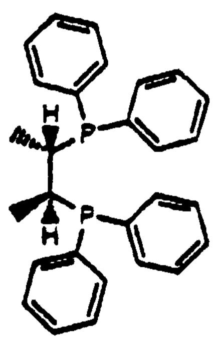 | (-) CHIRAPHOS<br>*(S,S)-2,3-bis(diphénylphosphino) butane.*<br>M = 426,48 | (-)CHIRAPHOS Ru (Mét.)$_2$<br>M = 635,74<br>(Jaune)<br>Rdt = 55% |
| Ex. 17 | 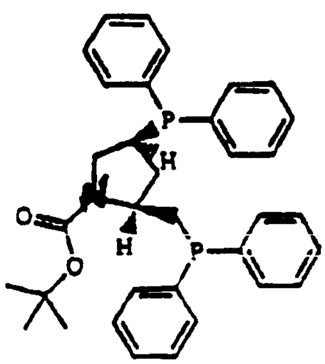 | (-) BPPM<br>*(2S, 4S) -N-BOC-4 diphénylphosphino-2-diphénylphosphino-2-méthylpyrrolidine*<br>M = 553,63 | (-) BPPM Ru (Mét.)$_2$<br>M = 762,88<br>(Vert pâle)<br>Rdt = 67 % |

Tableau II (fin)

| | LIGAND | | COMPLEXE |
|---|---|---|---|
| Ex. 18 | 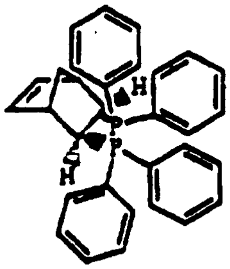 | (+) NORPHOS<br>*(S,S)-1,2-bis(diphénylphosphino) bicyclo [2.2.1] hept-4 ène.*<br>M = 462,61 | (+) NORPHOS Ru (Mét.)$_2$<br>M = 671,77<br>(Marron)<br>Rdt = 71% |
| Ex. 19 | 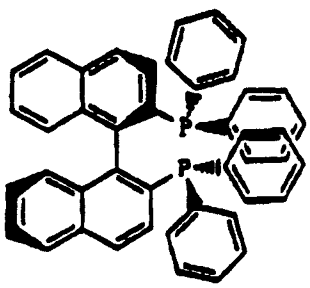 | (+) BINAP<br>*(R)-2,2'-bis(diphénylphosphino)-1,1'-binaphtyle*<br>M = 622,69 | (+) BINAP Ru (Mét.)$_2$<br>M = 831,95<br>(Marron)<br>Rdt = 32% |
| Ex. 20 | 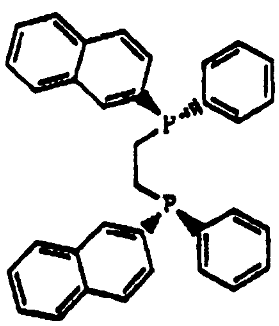 | (-) BNPE<br>*Bis (naphtyl phényl phosphino) éthane*<br>M = 498,54 | (-) BNPE Ru (Mét.)$_2$<br>M = 606,73<br>(Jaune vif)<br>Rdt = 43% |
| Ex. 21 | 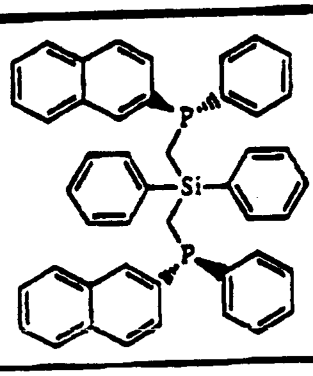 | "BNPPMDS"<br>*Bis (naphtylphényl-phosphino-méthano) diphénylsilane*<br>M = 684,88 | "BNPPMDS" Ru (Mét.)$_2$<br>M = 898,17<br>(Marron)<br>Rdt = 74% |

Ces complexes sont conservés sous atmosphère d'argon au réfrigérateur.

Exemples 22 à 29

On a étudié 8 des complexes chiraux du ruthénium figurant dans le tableau II ci-dessus (ceux des exemples 13 à 20) en tant que catalyseurs dans la préparation de l'acide tiglique par hydrogénation catalytique.

Dans cette optique, l'équation IV ci-après a été réalisée en utilisant 1 % de chaque complexe chiral dans du méthanol, avec de l'hydrogène sous une pression de 8,1 bars (8 atmosphères), à 25 °C pendant 24 h.

Les résultats obtenus ont été consignés dans le tableau III ci-après où Mét désigne le groupe méthallyle.

TABLEAU III

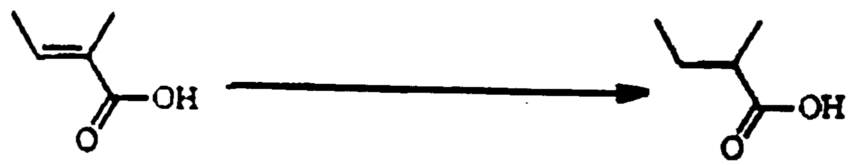

(IV)

| EXEMPLE | Catalyseur | Rendement %(1) |
|---|---|---|
| 22 | BDPP Ru (Mét.)$_2$ | 100 |
| 23 | DIOP Ru (Mét.)$_2$ | 100 |
| 24 | DIPAMP Ru (Mét.)$_2$ | 100 |
| 25 | CHIRAPHOS Ru (Mét.)$_2$ | 100 |
| 26 | BPPM Ru (Mét.)$_2$ | 100 |
| 27 | NORPHOS Ru (Mét.)$_2$ | 100 |
| 28 | BINAP Ru (Mét.)$_2$ | 100 |
| 29 | BNPE Ru (Mét.)$_2$ | 100 |

*(1) Déterminé par RMN du $^1H$.*

D'un point de vue pratique selon l'invention, l'on préfère faire appel à des complexes chiraux du ruthénium de formule (1) dans lesquels le groupe $R^1R^2P$-Q-$PR^3R^4$ comporte au total plus de 6 atomes de carbone, de préférence plus de 12 atomes de carbone et mieux de 24 à 50 atomes de carbone.

**Revendications**

1. Procédé d'hydrogénation de composés organiques éthylèniquement insaturés en milieu homogène, contenant un catalyseur formé par un complexe de ruthénium avec une phosphine, caractérisé en ce que le catalyseur employé comprend deux groupes allyliques en tant que ligands du Ru.

2. Procédé suivant la revendication 1, caractérisé en ce que les groupes allyliques sont choisis parmi les groupes allyle et méthallyle.

3. Procédé suivant la revendication 1 ou 2, caractérisé en ce que, dans le catalyseur utilisé, le ruthénium est complexé avec une diphosphine chirale, en particulier avec chiralité sur P.

4. Procédé suivant une des revendications précédentes, caractérisé en ce que le composé éthylèniquement insaturé est en solution, à raison de 0,1 à 3 moles par litre de solvant qui contient le catalyseur dans une proportion de 0,1 à 5 moles % par rapport au composé insaturé, et de préférence de 0,5 à 2 moles %.

**5.** Procédé suivant une des revendications précédentes, caractérisé en ce que l'hydrogénation est conduite sous une pression de $H_2$ de 1 à 100 bars, à une température de 0° à 50°C, de préférence en 24 à 64 heures.

**6.** Procédé suivant une des revendications précédentes, caractérisé en se que le milieu réactionnel contient également un catalyseur similaire mais non allylé, notamment halogéné ou carboxylé, dont la proportion préférée est de 10 à 90 parties en poids pour 90 à 10 parties de catalyseur allylé.

**7.** Procédé suivant la revendication 1, caractérisé en ce que ledit catalyseur répond à la formule schématique

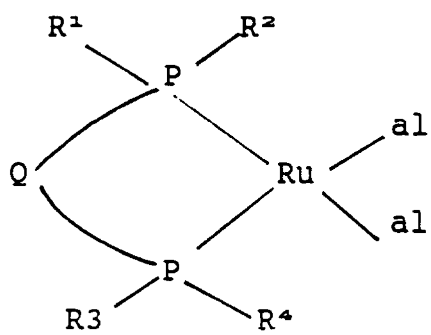

(1)

dans laquelle

| | |
|---|---|
| al | représente un groupe allylique, |
| Q | représente un pont hydrocarboné comportant au moins deux atomes de carbone caténaires et susceptible de contenir un à quatre hétéroatomes caténaires choisis parmi O, S, N et Si |
| $R^1$ à $R^4$ | qui peuvent être identiques ou différents, représentent chacun un groupe alkyle en $C_1$-$C_{18}$, cycloalkyle en $C_5$-$C_7$ ou aryle en $C_6$-$C_{12}$. |

**8.** Procédé suivant la revendication 7, caractérisé en ce que ledit pont Q est une chaîne comprenant de 2 à 10 atomes caténaires, un ou plusieurs fragments de cette chaîne pouvant être inclus dans au moins un cycle.

**9.** Procédé suivant la revendication 7, caractérisé en ce que le groupe $R^1R^2P$-Q-$PR^3R^4$ comporte au total plus de 6 atomes de carbone, de préférence plus de 12 atomes de carbone et mieux de 24 à 50 atomes de carbone.

**10.** Procédé suivant la revendication 7, caractérisé en ce que $R^1$, $R^2$, $R^3$ et $R^4$, identiques ou différents, représentent chacun un groupe cyclohexyle, phényle, phényle substitué en position para par un groupe alkyle en $C_1$-$C_4$, phényle substitué en position para par un groupe alkoxy en $C_1$-$C_4$, ou 2-naphtyle.

**11.** Complexe chiral du ruthénium, utile notamment en tant que catalyseur dans l'hydrogénation catalytique de composés organiques éthylèniquement insaturés, ledit complexe chiral étant caractérisé en ce qu'il répond à la formule schématique

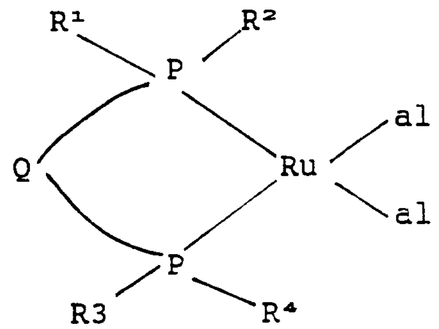

(1)

dans laquelle

al représente un groupe allylique,

Q représente un pont hydrocarboné comportant au moins deux atomes de carbone caténaires et susceptible de contenir un à quatre hétéroatomes caténaires choisis parmi O, S, N et Si

$R^1$ à $R^4$ qui peuvent être identiques ou différents, représentent chacun un groupe alkyle en $C_1$-$C_{18}$, cycloalkyle en $C_5$-$C_7$ ou aryle en $C_6$-$C_{12}$ ; et,

en ce que le groupe $R^1R^2P$-Q-$PR^3R^4$ comporte au total plus de 6 atomes de carbone, de préférence plus de 12 atomes de carbone et mieux de 24 à 50 atomes de carbone.

**12.** Complexe suivant la revendication 11, caractérisé en ce que le groupe $R^1R^2P$-Q-$PR^3R^4$ de sa molécule est constitué par une des diphosphines connues sous les abréviations "DIOP", "CHIRAPHOS", "NORPHOS", "BINAP", "DIPAMP", "BNPE", "CyDIOP", "CyPRONOP", "Cy-cyCAPP", "CyPOP AE", "BDPP", "BPPM" ou "BNPPDS".

**13.** Application du procédé suivant l'une quelconque des revendications 1 à 10, à l'hydrogénation de la double liaison C=C de composés oléfiniques.

**14.** Application suivant la revendication 13, caractérisée en ce que les composés à hydrogéner portent un ou plusieurs groupes fonctionnels, en particulier des groupes carboxyle ou/et acylamido.

## Claims

**1.** A process for the hydrogenation of ethylenically unsaturated organic compounds in a homogeneous medium containing a catalyst formed by a complex of ruthenium with a phosphine, wherein the catalyst employed comprises two allylic groups as ligands on the Ru.

**2.** A process according to claim 1 wherein the allylic groups are selected from allyl and methallyl groups.

**3.** A process according to claim 1 or 2 wherein, in the catalyst used, the ruthenium is complexed with a chiral diphosphine, in particular a diphosphine with chirality on the P.

**4.** A process according to one of the preceding claims wherein the ethylenically unsaturated compound is in solution at a rate of 0.1 to 3 mol per liter of solvent, which contains the catalyst in a proportion of 0.1 to 5 mol%, based on the unsaturated compound, and preferably of 0.5 to 2 mol%.

**5.** A process according to one of the preceding claims wherein the hydrogenation is carried out under an $H_2$ pressure of 1 to 100 bar, at a temperature of 0° to 50°C, preferably in 24 to 64 hours.

**6.** A process according to one of the preceding claims wherein the reaction medium also contains a similar but non-allylated catalyst, especially a halogenated or carboxylated catalyst, the preferred proportion of which is 10 to 90 parts by weight to 90 to 10 parts of allylated catalyst.

7. A process according to claim 1 wherein said catalyst has the schematic formula

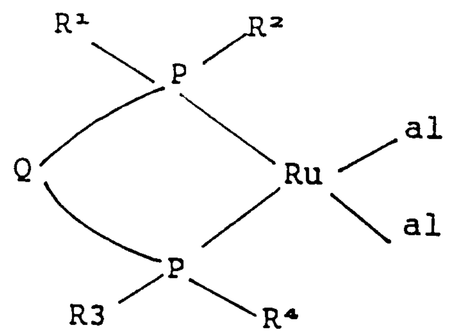

(1)

in which

| | |
|---|---|
| al | is an allylic group, |
| Q | is a hydrocarbon bridge containing at least two catenary carbon atoms and capable of containing one to four catenary heteroatoms selected from O, S, N and Si, and |
| $R^1$ to $R^4$ | can be identical or different and are each a $C_1$-$C_{18}$-alkyl, $C_5$-$C_7$-cycloalkyl or $C_6$-$C_{12}$-aryl group. |

8. A process according to claim 7 wherein said bridge Q is a chain comprising from 2 to 10 catenary atoms, it being possible for one or more fragments of this chain to be included in at least one ring.

9. A process according to claim 7 wherein the group $R^1R^2P$-Q-$PR^3R^4$ contains a total of more than 6 carbon atoms, preferably more than 12 carbon atoms and particularly preferably from 24 to 50 carbon atoms.

10. A process according to claim 7 wherein $R^1$, $R^2$, $R^3$ and $R^4$, which are identical or different, are each a cyclohexyl group, a phenyl group, a phenyl group substituted in the para-position by a $C_1$-$C_4$-alkyl group, a phenyl group substituted in the para-position by a $C_1$-$C_4$-alkoxy group, or a 2-naphthyl group.

11. A chiral ruthenium complex useful especially as a catalyst in the catalytic hydrogenation of ethylenically unsaturated organic compounds, said chiral complex having the schematic formula

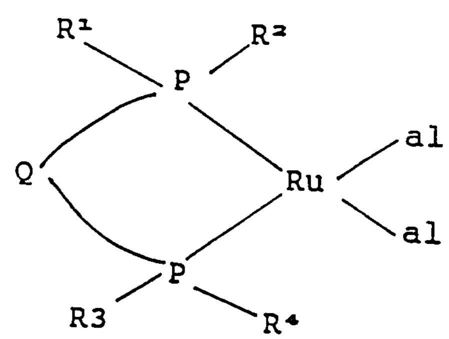

(1)

in which

| | |
|---|---|
| al | is an allylic group, |
| Q | is a hydrocarbon bridge containing at least two catenary carbon atoms and capable of containing one to four catenary heteroatoms selected from O, S, N and Si, and |
| $R^1$ to $R^4$ | can be identical or different and are each a $C_1$-$C_{18}$-alkyl, $C_5$-$C_7$-cycloalkyl or $C_6$-$C_{12}$-aryl group, and |

the group $R^1R^2P$-Q-$PR^3R^4$ containing a total of more than 6 carbon atoms, preferably more than 12 carbon atoms and particularly preferably from 24 to 50 carbon atoms.

12. A complex according to claim 11 wherein the group $R^1R^2P\text{-}Q\text{-}PR^3R^4$ in its molecule consists of one of the diphosphines known by the abbreviations "DIOP", "CHIRAPHOS", "NORPHOS", "BINAP", "DIPAMP", "BNPE", "CyDIOP", "CyPRONOP", "Cy-cyCAPP", "CyPOP AE", "BDPP", "BPPM" and "BNPPDS".

13. Application of the process according to any one of claims 1 to 10 to the hydrogenation of the C=C double bond of olefinic compounds.

14. Application according to claim 13 wherein the compounds to be hydrogenated carry one or more functional groups, in particular carboxyl and/or acylamido groups.

**Patentansprüche**

1. Verfahren zur Hydrierung von ethylenisch ungesättigten organischen Verbindungen in einem homogenen Milieu, das einen Katalysator enthält, der aus einem Komplex von Ruthenium mit einem Phosphin gebildet ist, dadurch gekennzeichnet, daß der verwendete Katalysator zwei Allylgruppen als Ru-Liganden umfaßt.

2. Verfahren nach Anspruch 1, dadurch gekennzeichnet, daß die Allylgruppen ausgewählt sind aus den Gruppen mit Allyl und Methallyl.

3. Verfahren nach Anspruch 1 oder 2, dadurch gekennzeichnet, daß im verwendeten Katalysator das Ruthenium mit einem chiralen Diphosphin, insbesondere mit einer Chiralität an P, komplexiert gebunden ist.

4. Verfahren nach einem der vorhergehenden Ansprüche, dadurch gekennzeichnet, daß die ethylenisch ungesättigte Verbindung in Lösung in einer Menge von 0.1 bis 3 Mol pro Liter Lösungsmittel vorliegt, das den Katalysator in einer Menge von 0.1 bis 5 Mol %, bezogen auf die ungesättigte Verbindung, und vorzugsweise in einer Menge von 0.5 bis 2 Mol-%, enthält.

5. Verfahren nach einem der vorhergehenden Ansprüche, dadurch gekennzeichnet, daß die Hydrierung unter einem $H_2$-Druck von 1 bis 100 Bar bei einer Temperatur von 0 bis 50 °C, und vorzugsweise während 24 bis 64 Stunden, durchgeführt wird.

6. Verfahren nach einem der vorhergehenden Ansprüche, dadurch gekennzeichnet, daß das Reaktionsmilieu außerdem einen ähnlichen, aber nicht allylierten, und insbesondere halogenierten oder carboxylierten, Katalysator in einem Verhältnis von vorzugsweise 10 bis 90 Gewichtsteilen pro 90 bis 10 Gewichtsteilen des allylierten Katalysators enthält.

7. Verfahren nach Anspruch 1, dadurch gekennzeichnet, daß der Katalysator der allgemeinen Formel (I) entspricht

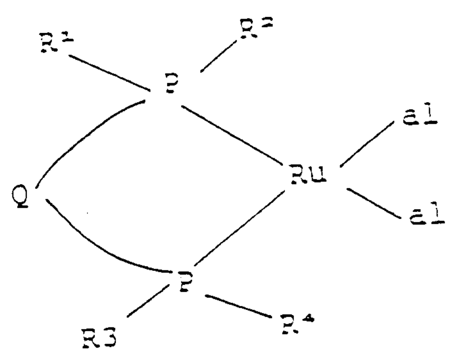

(I)

worin bedeuten
al eine Allylgruppe
Q eine Kohlenwasserstoffbrücke, die mindestens 2 Ketten-Kohlenstoffatome enthält und 1 bis 4 Ketten-Heteroatome enthalten kann, ausgewählt aus O, S, N und Si;
R1 und R4, die gleich oder verschieden sein können, eine C1-C18 Alkylgruppe, eine C5-C7-Cycloalkylgruppe oder eine C6-C12-Arylgruppe.

**8.** Verfahren nach Anspruch 7, dadurch gekennzeichnet, daß die Brücke Q eine Kette ist, die 2 bis 10 Kettenatome enthält, wobei eine oder mehrere Fragmente dieser Kette in mindestens einem Ring enthalten sein können.

**9.** Verfahren nach Anspruch 7, dadurch gekennzeichnet, daß die Gruppe R1R2P-Q-PR3R4 insgesamt mehr als 6 Kohlenstoffatome enthält, vorzugsweise mehr als 12 Kohlenstoffatome, und in erster Linie 24 bis 50 Kohlenstoffatome.

**10.** Verfahren nach Anspruch 7, dadurch gekennzeichnet, daß R1, R2, R3 und R4, die gleich oder verschieden sind, eine Cyclohexylgruppe, Phenyl, in para-Stellung durch eine C1-C4-Alkylgruppe substituiertes Phenyl, in para-Stellung durch eine C1-C4-Alkoxygruppe substituierte Phenylgruppe, oder Naphtyl-(2) ist.

**11.** Chiraler Rutheniunkomplex, insbesondere zur Verwendung als Katalysator bei der katalytischen Hydrierung von ethylenisch ungesättigten organischen Verbindungen, dadurch gekennzeichnet, daß der chirale Komplex der allgemeinen Formel

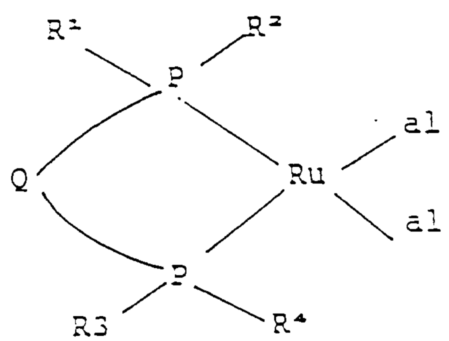

(I)

entspricht, worin bedeuten
al eine Allylgruppe
Q eine Kohlenwasserstoffbrücke, die mindestens 2 Ketten-Kohlenstoffatome enthält und 1 bis 4 Ketten-Heteroatome enthalten kann, ausgewählt aus O, S, N und Si;
R1 und R4, die gleich oder verschieden sein können, eine C1-C18-Alkylgruppe, eine C5-C7-Cycloalkylgruppe oder eine C6-C12-Arylgruppe bedeuten; und
dadurch, daß die Gruppe R1R2P-Q-PR3R4 insgesamt mehr als 6 Kohlenstoffatome, vorzugsweise mehr als 12 Kohlenstoffatome und insbesondere 24 bis 50 Kohlenstoffatome umfaßt.

**12.** Komplex nach Anspruch 11, dadurch gekennzeichnet, daß die Gruppe R1R2P-Q-PR3R4 im Molekül eines der Diphosphine ist, die unter den Abkürzungen "DIOP", "CHIRAPHOS", "NORPHOS", "BINAP", "DIPAMP", "BNPE", "CyDIOP", "CyPRONOP", "Cy-cyCAPP", "CyPOP AE", "BDPP", "BPPM" oder "BNPPDS" bekannt sind.

**13.** Verwendung eines Verfahrens nach einem der Ansprüche 1 bis 10 zur Hydrierung einer C=C-Doppelbindung von olefinischen Verbindungen.

**14.** Verwendung nach Anspruch 13, dadurch gekennzeichnet, daß die zu hydrierenden Verbindungen eine oder mehrere funktionellen Gruppen enthalten, und insbesondere die Gruppen Carboxyl und/oder Acylamido.